(19) Europäisches Patentamt
European Patent Office
Office européen des brevets

(11) Publication number: **0 302 372 B1**

(12) # EUROPEAN PATENT SPECIFICATION

(45) Date of publication of patent specification: **16.12.92**

(51) Int. Cl.⁵: **A61K 31/435**, //C07D471/04

(21) Application number: **88112104.0**

(22) Date of filing: **27.07.88**

(54) **Naphthyridine antianaerobic compounds.**

(30) Priority: **04.08.87 US 81413**

(43) Date of publication of application:
**08.02.89 Bulletin 89/06**

(45) Publication of the grant of the patent:
**16.12.92 Bulletin 92/51**

(84) Designated Contracting States:
**AT BE CH DE FR GB IT LI LU NL SE**

(56) References cited:
**EP-A- 0 153 580**
**DE-A- 3 542 972**
**GB-A- 2 170 804**
**DE-A- 3 514 076**
**DE-A- 3 632 222**
**JP-B- 6 237 006**

**Journal of Antimicrobial Chemotherapy (1986)( 18, suppl. D, pp. 1-20, King et al.**

**Antimicrobial Agents and Chemotherapy, 32, Nr. 1, Jan. 1988, pp. 27-32, Fernandes et al.**

**PATENT ABSTRACTS OF JAPAN, unexamined applications, section C, vol. 10, no. 328, November 7, 1986 THE PATENT OFFICE JAPANESE GOVERNMENT, pp. 154 C 383**

(73) Proprietor: **ABBOTT LABORATORIES**

**Abbott Park, Illinois 60064(US)**

(72) Inventor: **Fernandes, Prabhavathi**
**1151 Polo Drive**
**Lake Forest Illinois 60045(US)**
Inventor: **Chu, Daniel Tim-Wo**
**204 Ashland Court**
**Vernon Hills Illinois 60061(US)**

(74) Representative: **Modiano, Guido et al**
**Modiano & Associati S.r.l. Baaderstrasse 3**
**W-8000 München 5(DE)**

EP 0 302 372 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a new use of naphthyridine derivatives. These derivatives have unexpected and superior antibacterial properties against anaerobic bacteria.

It is known that certain naphthyridine compounds exhibit antibacterial properties, most notably Enoxacin which is an 1-ethyl-6-fluoro-1,4-dihydro-4-oxo 7-piperazin-1-yl,8-naphthyridine-3-carboxylic acid. However, this compound and other quinoline analogs are not very effective against anaerobic organisms.

This invention relates to a new use of 1-o,p-difluorophenyl-6-fluoro-1,4-dihydro-4-oxo-7-(3-aminopyrrolidin-1-yl)-1,8-naphthyridine-3-carboxylic acid and derivatives thereof having the formula I:

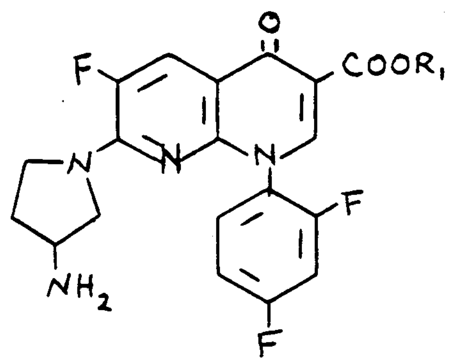

(I)

wherein $R_1$ is a hydrogen atom or a carboxy-protecting group; and pharmaceutically acceptable salts thereof.

As used herein, the term "carbon-protecting group" refers to a carboxy group which has been esterified with one of the commonly used carboxylic acid protecting ester groups employed to block or protect the carboxylic acid functionality while reactions involving other functional sites of the compound are carried out. In addition, a carboxy protecting group can be used as a prodrug whereby the carboxy protecting group can be hydrolyzed enzymatically to release the biologically active parent acid. Such protected carboxy groups are noted for their ease of cleavage by hydrolytic methods to the corresponding carboxylic acid. Further, such carbon-protecting groups can be relatively easily cleaved to yield the corresponding free carboxy group. Such carboxy-protecting groups are well known to those skilled in the art, having been extensively used in the protection of carboxyl groups in the penicillin and cephalosporin fields, as described in U.S. Patent Nos. 3,840,556 and 3,719,667. Representative protecting groups include $C_1$ to $C_8$ alkyl (e.g., methyl, ethyl, tertiary butyl), benzyl and substituted derivatives thereof such as alkoxy and nitrobenzyl groups, dialkylaminoalkyl (e.g. dimethylaminoethyl), acyloxyalkyl groups such as pivaloyloxymethyl and propionyloxy methyl.

The chiral centers of the compounds of the invention may have either the "R" or "S" configuration.

Also included within the scope of the present invention is the use of pharmaceutically acceptable salts of the foregoing compounds. As used herein, the term "pharmaceutically acceptable salts" refers to non-toxic acid addition salts and alkaline earth metal salts of the compounds of formula I. The salts can be prepared in situ during the final isolation and purification of the compounds of formula I, or separately by reacting the free base or acid functions with a suitable organic acid or base. Representative acid addition salts include the hydrochloride, hydrobromide, sulphate, bisulphate, acetate, oxalate, valerate, oleate, palmitate, stearate, laurate, borate, benzoate, lactate, phosphate, tosylate, mesylate, citrate, maleate, fumarate, succinate, tartrate, glucoheptonate, lactobionate and lauryl sulfate salts. Representative alkali or alkaline earth metal salts include the sodium, calcium, potassium and magnesium salts.

It has been found that the compounds of formula I possess antibacterial activity against a wide spectrum of gram positive and gram negative anaerobic bacteria. The compounds of formula I are therefore useful in the antibiotic treatment of susceptible bacterial infections in both humans and animals.

Representative of the preferred compounds to be used according to the invention include 1-o,p-difluorophenyl-6-fluoro-1,4-dihydro-4-oxo-7-(3-aminopyrrolidin-1-yl)-1,8-naphthyridine-3-carboxylic acid hydrochloride, 1-o,p-difluorophenyl-6-fluoro-1,4-dihydro-4-oxo-7-(3-aminopyrrolidin-1-yl)-1,8-naphthyridine-3-carboxylic acid sulfate, 1-o,p-difluorophenyl-6-fluoro-1,4-dihydro-4-oxo-7-(3-aminopyrrolidin-1-yl)-1,8-naphthyridine-3-carborylic acid tosylate.

Susceptible organisms generally include those gram positive and gram negative anaerobic organisms whose growth can be inhibited by the compounds of formula I such as Bacteroides fragilis, other

Bacteroides species, Fusobacterium nucleatum, other Fusobacterium species, Veillonella, parvula, Clostridium difficile, Clostridium perfringen, other Clostridium species, peptococci and peptostreptococci and Propionibacterium acnes.

The compounds of formula I may also be formulated into compositions together with pharmaceutically acceptable carriers for parenteral injection, for oral administration in solid or liquid form, for rectal administration, and the like.

Compositions for the new use according to the invention for parenteral injection may comprise pharmaceutically acceptable sterile aqueous or nonaqueous solutions, suspensions or emulsions. Examples of suitable nonaqueous carriers, diluents, solvents or vehicles include propylene glycol, polyethylene glycol, vegetable oils, such as olive oil, and injectable organic esters such as ethyl oleate. Such compositions may also contain adjuvants such as preserving, wetting, emulsifying, and dispersing agents. They may be sterilized, for example, by filtration through a bacteria-retaining filter, or by incorporating sterilizing agents into the compositions. They can also be manufactured in the form of sterile solid compositions which can be dissolved in sterile water, or some other sterile injectable medium immediately before use.

Solid dosage forms for oral administration include capsules, tablets, pills, powders and granules. In such solid dosage forms, the active compound is admixed with at least one inert diluent such as sucrose, lactose or starch. Such dosage forms can also comprise, as is normal practice, additional substances other than diluents, e.g., lubricating agents such as magnesium stearate. In the case of capsules, tablets and pills, the dosage forms may also comprise buffering agents. Tablets and pills can additionally be prepared with enteric coatings.

Liquid dosage forms for oral administration include pharmaceutically acceptable emulsions, solutions, suspensions, syrups and elixers containing inert diluents commonly used in the art, such as water. Besides such inert diluents, compositions can also include adjuvants, such as wetting agents, emulsifying and suspending agents, and sweetening, flavoring and perfuming agents.

Compositions for rectal administration are preferably suppositories which may contain, in addition to the active substance, excipients such as cocoa butter or a suppository wax.

Actual dosage levels of active ingredient in the above compositions may be varied so as to obtain an amount of active ingredient effective to achieve antibacterial activity in accordance with the desired method of administration. The selected dosage level therefore depends upon the nature of the active compound administered, the route of administration, the desired duration of treatment and other factors. Generally, daily dosage levels of the compounds of formula I of about 0.1 to about 750, more preferably about 0.25 to about 250 and most preferably about 0.5 to about 30 mg. of active ingredient per kg. of body weight are effective when administered orally to a mammalian patient suffering from an infection caused by a susceptible organism. If desired, the daily dose may be divided into multiple doses for administration, e.g., two to four times per day.

Compounds for the new use according to this invention can be prepared by the reaction illustrated below:

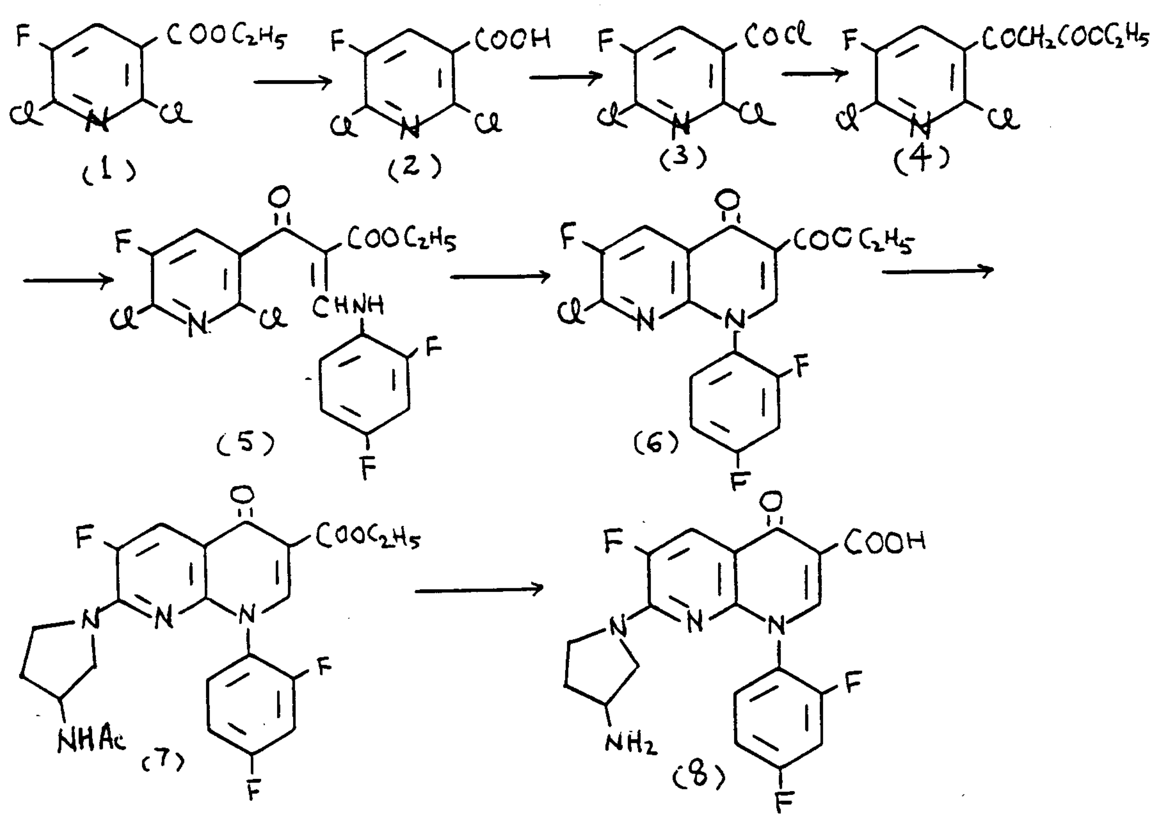

(1) (2) (3) (4) (5) (6) (7) (8)

Hydrolysis of ethyl 2,6-dichloro-5-fluoronicotinate (1) with 6 N hydrochloric acid in trifluoroacetic acid yielded the 2,6-dichloro-5-fluoronicotinic acid (2). Treatment of (2) with thionyl chloride gave the 2,6-dichloro-5-fluoro-nicotinyl chloride (3). Reaction of the acid chloride (3) with dilithio dianion of monoethyl malonate afforded the ethyl 2,6-dichloro-5-fluoronicotinyl acetate (4). Treatment of this ester with triethyl orthoformate in acetic anhydride gave the one-carbon homologue enol ether intermediate, which upon evaporation of solvent to dryness was allowed to react with a slight excess of an appropriate aniline in methylene chloride at room temperature to give the ethyl 3-anilino-2-(2,6-dichloro-5-fluoro)nicotinyl-acrylates (5). Cyclization of compounds with 1 M equivalent of sodium hydride in tetrahydrofuran (THF) yielded ethyl 1,4-dihydro-4-oxo-1,8-naphthyridine-3-carboxylates (6). Displacement of the 7-chlorine atom of the carboxylates (6) with an appropriate amine in methylene chloride or pyridine yielded the desired 7-amino derivatives (7). Hydolysis of the ethyl ester (7) with hydrochloric acid gave the desired 7-substituted amino-6-fluoro-1-aryl-1,4-dihydro-4-oxo-1,8-naphthyridine-3-carboxylic acids (8).

The foregoing may be better understood from the following examples, which are presented for purposes of illustration.

Example 1

1-o,p-Difluorophenyl-6-fluoro-1,4-dihydro-4-oxo-7-(3-amino pyrrolidin-1-yl)-1,8-naphthyridine-3-carboxylic acid hydrochloride salt

Ethyl 2,6-dichloro-5-fluoronicotinate (20 g, 84 mmol) was dissolved in a mixture of 40 ml of trifluoroacetic acid and 40 ml of 7.5 N HCl. The mixture was heated to reflux for 24 hours. The solution was cooled, and the trifluoroacetic acid was removed by evaporation under reduced pressure. Upon cooling, 100 ml of water was added and a white precipitate formed. The precipitate was filtered, washed with hexane, and dried, yielding 12.91 g (73.2%) of 2,6-dichloro-5-fluoronicotinic acid: mp 153-154 °C.

2,6-Dichloro-5-fluoronicotinic acid (7 g, 33.3 mmol) was dissolved in thionyl chloride (35 ml). After the mixture was heated at 80 °C for 2 h, the thionyl chloride was removed by evaporation under reduced pressure yielding a yellowish 2,6-dichloro-5-fluoronicotinyl chloride. Monoethyl malonate (13.2 g, 100 mmol) and 3 mg of biquinoline was dissolved in 280 ml of dry tetrahydrofuran (THF) and cooled to -30 °C. A solution of 2.5 M n-butyllithium in hexane was added until a pink color remained at -5 °C (80 ml). The suspension was then cooled to -50 °C. The acid chloride obtained as described above, dissolved in 50 ml of

THF, was then added to the suspension dropwise. After the addition, the dry ice bath was removed and the reaction was allowed to stir at room temperature for 1 hour. The reaction was acidified with 200 ml of 1 N HCl and was extracted with ether. The ether fraction was washed with saturated aqueous sodium bicarbonate solution and then water. The ether solution was dried and evaporated to dryness yielding a residue, which was washed with hexane to give 9.14 g (97.9%) (mp 64-65 ° C) of ethyl 2,6-dichloro-5-fluoronicotinylacetate.

A solution of ethyl 2,6-dichloro-5-fluoronicotinylacetate (7.25 g, 26 mmol) in triethyl orthoformate (5.9 g, 40 mmol) and acetic anhydride (10.9 g, 107 mmol) was heated at 130 ° C for 1 hour with removal of ethyl acetate formed during the reaction. The solution was evaporated under reduced pressure to a mobile oil, which was then dissolved in methylene chloride (70 ml). 2,4-Difluoroaniline (3.69 g, 28.6 mmol) in 10 ml methylene chloride was added to the solution. After 0.5 hour, the solution was evaporated to dryness and the residue crystallized and washed with hexane, yielding 8.77 g (81.4%) of nicotinylacrylate derivative (mp 138-140 ° C).

To a solution of the above nicotinylacrylate (8.70 g, 20.7 mmol) in THF (20 ml) at ice bath was added in a 60% sodium hydride is oil suspension (0.87 g). The ice bath was removed and the mixture was heated at reflux under nitrogen atmosphere for 1 hour and was cooled and poured into ice water. The precipitated was filtered and washed with water. The residue was triturated in 50% ether and 50% hexane solution yielding 5.63 g (71.0%) of ethyl 1-o,p-difluorophenyl-6-fluoro-7-chloro-1,4-dihydro-4-oxo-1,8-naphthyridine-3-carboxylate (mp 211-212 ° C).

To a solution of the carboxylate (1.68 g, 4.4 mmol) in methylene chloride (50 ml) was added 3-acetamidopyrrolidine (3.7 g), and the mixture was stirred at room temperature for 8 hours. The solvent was removed under reduced pressure, and the residue was partitioned between methylene chloride and water. The organic layer was dried and evaporated under reduced pressure to give a solid. This was suspended in water and stirred for 18 hours. The sold was filtered and dried, yielding 1.69 g (81.2%) of ethyl 1-o,p-difluorophenyl-6-fluoro-7-(3-acetamidopyrrolidin-1-yl)-1,4-dihydro-4-oxo-1,8-naphthyridine-3-carboxylate (mp 227-229 ° C).

A suspension of the above carboxylate (3.4 g, 7.4 mmol) in 6N HCl solution (20 ml) was heated at 110 ° C for 24 hours. The mixture was evaporated to dryness under reduced pressure. The residue was washed with absolute ethanol and filtered yielding 1-o,p-difluorophenyl-6-fluoro-1,4-dihydro-4-oxo-7-(3-aminopyrrolidin-1-yl)-1,8-naphthyridine-3-carboxylic acid hydrochloride salt.

IR (KBr) $cm^{-1}$; CO 1730
NMR (DMSO-$D_6$); delta value
2.11 (2H, m),
3.82 (5H, m),
7.36 (1H, m),
7.60 (1H, m),
7.81 (1H, m),
8.13 (1H, d, J = 13 Hz)
8.27 (3H, sb),
8.84 (1H, s)
mp >250 ° C

Example 2

1-o,p-Difluorophenyl-6-fluoro-1,4-dihydro-4-oxo-7-(3-aminopyrrolidin-1-yl)-1,8-naphthyridine-3-carboxylic acid

2.5 g of the product of Example 1 was dissolved in 17 ml of 1 N sodium hydroxide solution. Water (60 ml) was added and then followed by the addition of acetic acid (0.7 ml). The precipitate was filtered and washed with water yielding 2.2 g of 1-o,p-difluorophenyl-6-fluoro-1,4-dihydro-4-oxo-7-(3-aminopyrrolidinyl-1-yl)-1,8-naphthyridine-3-carboxylic acid (96%).

IR (HBr) $cm^{-1}$; CO 1725
NMR (DMSO-$D_6$); delta value
1.65 (1H, m),
1.97 (1H, m),
3.55 (5H, m),
7.27 (1H, m),
7.45 (1H, m)
7.75 (1H, m),

7.99 (1H, d, J = 13 Hz),
8.69 (1H, s),
mp 247-250 ° C

Example 3

Sulfate salt of Example 2

To a suspension of product of Example 2 (404 mg, 1 mmol) in water (35 ml) was added in 1 ml 1 N sulfuric acid solution. The mixture was warm to dissolve and evaporated under reduced pressure to dryness to give the sulfate salt of Example 2 (430 mg).
IR (HBr) $cm^{-1}$; CO 1725
NMR (DMSO-$D_6$); delta value
2.01 (1H, m),
2.19 (1H, m),
3.26-3.86 (5H, m),
4.01 (1H, m),
7.36 (1H, m),
7.60 (1H, m),
7.81 (1H, m),
8.15 (1H, d, J = 13 Hz),
8.85 (1H, s),

Example 4

Tosylate salt of Example 2

A suspension of 1.02 g of Example 2 and 480 mg of p-toluenesulfonic acid monohydrate in 100 ml of water was heated to boiling until it became a solution. The solution was then cooled to room temperature and the product was then freeze dried to yield the tosylate salt of Example 2 (1.40 g).
IR (KBr) $cm^{-1}$; CO 1725
NMR (DMSO-$D_6$); delta value
2.28 (3H, s),
3.2-3.9 (7H, m),
7.11 (2H, d, J = 8 Hz),
7.35 (1H, m),
7.47 (2H, d, J = 8 Hz,)
7.59 (1H, m),
7.82 (1H, m),
7.95 (2H, m),
8.14 (1H, d, J = 13 Hz),
8.86 (1H, s),

Example 5

Methanesulfonate salt of Example 2

To a suspension of product of Example 2 (404 mg) in water (40 ml) was added in 97 mg of methanesulfonic acid. The mixture was warm to dissolve and the solution was freeze dried to give the methanesulfonate salt of Example 2 (500 mg).
IR (KBr) $cm^{-1}$; CO 1725
NMR (DMSO-$D_6$); delta value
2.03 (1H, s),
2.20 (1H, m),
2.30 (3H, s),
3.21-3.82 (5H, m),
7.35 (1H, m),
7.58 (1H, m),

7.83 (1H, m),
7.95 (2H, m),
8.15 (1H, d, J = 13 Hz),
8.84 (1H, s).

Example 6

**(s)-7-(3-Aminopyrrolidin-1-yl)-1-(o,p-difluorophenyl)-6-fluoro-1,4-dihydro-4-oxo-1,8-naphthyridine-3-carboxylic acid hydrochloride salt**

(a) Under a nitrogen atmosphere, in a 25 ml round-bottom flask were placed 120 mg (0.94 mmol) of (3s)-3-acetamidopyrrolidine (which can be made as in Example 7), 0.3 ml of pyridine and 0.17 ml (120 mg, 1.2 mmol) of triethylamine. To the system was added 430 mg (1.13 mmol) of ethyl 1-o,p-difluorophenyl-6-fluoro-7-chloro 1,4-dihydro-4-oxo-1,8-naphthyridine-3-carboxylate, and the reaction mixture was heated at 65 ° C for 21 h and concentrated with a rotary evaporator. The crude product was purified by flash column chromatography (180 g of silica gel), gradually increasing the polarity of the eluent from chloroform to 5% methanol/chloroform to obtain 343 mg of (s)ethyl 7-(3-acetamidopyrrolidin-1-yl-nyl)-1-(o,p-difluorophenyl)-6-fluoro-1,4-dihydro-4-oxo-1,8-naphthyridine-3-carboxylate as an orange solid, mp 240-243 ° C.

(b) Under a nitrogen atmosphere, in a 100 ml round-bottom flask were placed 223 mg (0.47 mmol) of the above carboxylate and 3ml of THF. To the system was added 95 ml of 0.1M aqueous sodium hydroxide. The reaction mixture was heated at 65 ° C for 2 hours and concentrated with a rotary evaporator. The residue was dissolved in water and the pH was adjusted to 4 kg addition of acetic acid. The precipitate was filtered. The residue was suspended in 10 ml 6M aqueous hydrochloric acid and the mixture was heated to reflux. After 19h, the mixture was concentrated and cooled, and the resulting off-white solid, (s)-7-(3-aminopyrrolidin-1-yl)-1-(o,p-difluorophenyl)-6-fluoro-1,4-dihydro-4-oxo-1,8-naphthyridine-3-carboxylic acid hydrochloride salt, was collected and dried in a vacuum oven (101 mg).

NMR (DMSO-$D_6$); delta value
2.00-3.90 (complex)
7.38 (1H, m)
7.57 (1H, m)
7.83 (1H, m)
8.13 (1H, d), J = 14 Hz)
8.83 (1H, s)

Example 7

(3s)-3-Acetamidopyrrolidine

A system protected from moisture was charged with 100 g (0.37 mol) of N-carbobenzyloxy-L-aspartic acid suspended in 125 ml (1.13 mol) of acetic anhydride. The reaction mixture was heated at 130 ° C until homogeneous (5-10 min), cooled to room temperature and diluted with 750 ml of ether. Hexane was then added with stirring until the reaction mixture was turbid. The product was allowed to stand overnight at room temperature. The product was isolated by suction filtration and the filter cake was washed with hexane 4x900 ml. A white solid was dried at 50 ° C under vacuum giving 80g (87%) of N-carbobenzyloxy-L-aspartic anhydride, mp 102-104 ° C. Mass Spectrum, M/Z 249.

NMR (DMSO-$D_6$); delta value
3.0 (d, 1H, J = 6.4Hz)
3.3 (d, 1H, J = 7.1Hz)
4.8 (m, 1H, J = 6.4Hz)
5.1 (s, 2H)
7.35 (s, 5H)
8.2 (d, 1H, J = 7.1Hz)

A system protected from moisture was charged with 80g (0.32 mol) of N-carbobenzyloxy-L-aspartic anhydride in 500 ml of absolute ethanol. To this was added dropwise with stirring 103 ml (0.94mol) of benzylamine in 100 ml of absolute ethanol. The addition was exothermic and the reaction mixture became homogeneous during the addition of benzylamine. After the addition was complete the reaction mixture was stirred at room temperature of 18 h. The reaction mixture was acidified to pH 3 with 1N HCl. The resulting

precipitate was isolated by suction filtration and was washed with $H_2O$ 3x250 ml and was dried under vacuum at 40° C. The product was a mixture of amide isomers. The product was a white solid 81g (71%), mp 124-134° C. Mass Spectrum, M/Z 356.

NMR (DMSO-$D_6$); delta value
2.6-2.8 (m, 2H)
4.3 (d, 2H, J = 5.7Hz)
4.4 (m, 1H)
5.1 (s, 2H)
7.2 (m, 11H)
8.5 (m, 1H)

An oven-dried system protected from moisture was charged with 80g (0.227 mol) of the mixture of amide isomers from the previous step suspended in 150 ml (1.59 mol) of acetic anhydride. The reaction mixture was heated at 55-60° C for 24 h. Cooled to room temperature, the solvent was removed on a rotary evaporator. The residue was dissolved in 1 liter of $CH_2Cl_2$ and was washed with 500 ml of 10% $Na_2CO_3$ and 500 ml $H_2O$. The organic layer was dried over $MgSO_4$, filtered, and the solvent removed on a rotary evaporator. The product was recrystallized from 95% ethanol, giving a white solid after drying under vacuum 65g (85%), mp 136-137° C. Mass Spectrum, m/z 338.

NMR ($CDCl_3$); delta value
2.60 (dd, 1H, J = 7.1Hz, 17.5Hz)
3.05 (d,d, 1H, J = 8.5Hz)
4.25 (m, 1H, J = 7.1 Hz)
4.65 (s, 2H)
5.05 (s, 2H)
5.65 (d, 1H, J = 7.1Hz)
7.30 (m, 10H)

The imide from the previous step 11.6g (34 mmol) dissolved in 250 ml of MeOH was treated with 1.2g of 20% Pd/C at room temperature for 18h under a hydrogen atmosphere. The catalyst was removed by filtration and was washed with 250 ml of MeOH. The solvent was removed on a rotary evaporator. After drying under vacuum the crude product was purified by chromatography on 200 g of 70-230 mesh silica gel. The column was eluted with 5% MeOH/$CH_2Cl_2$. Pooling of similar fractions and removal of the solvent gave a white solid N-benzyl-3S-aminosuccinimide 5.4g (76%), mp 70-71° C. Mass Spectrum, m/z 204.

NMR ($CDCl_3$); delta value
1.75 (bs, 2H)
2.40 (d,d, 1H, J = 17.8Hz, 5.7Hz)
3.00 (d,d, 1H, J = 17.8Hz, J = 8.5Hz)
3.85 (ddd, 1H, J = 17.8Hz, J = 5.7Hz, J = 85Hz)
4.15 (s, 2H)
7.40 (m, 5H)

An oven-dried system under positive $N_2$ atmosphere was charged with 230 mg (6.2 mmol) of $LiAlH_4$ suspended in 7 ml of dry THF, cooled in an ice bath and added 1.15g (5.6 mml) of N-benzyl-3s-aminosuccinimide suspended in 20 ml of dry THF dropwise with stirring. After the solution was complete, the reaction mixture was stirred at room temperature for 18h, cooled in an ice bath and 230 ml of $H_2O$, 230 ml 6N NaOH and 69 ml of $H_2O$ was cautiously added. The resulting precipitate was removed by suction filtration and was washed with EtOAc 3 x 50 ml. The combined organic layers were dried over $Na_2SO_4$. The drying agent was removed by filtration and the solvent was removed on a rotary evaporator giving 860 mg (87%) of a clear colorless oil. The oil was dissolved in 5ml of dry benzene and was placed in an oven-dried flask protected from moisture. To this was added 555 ml (5.0 mmol) of acetic anhydride in 2 ml of dry benzene. The reaction mixture was stirred at room temperature for 20 h. The reaction mixture was neutralized with 0.5N NaOH. The aqueous layer was extracted with $CH_2Cl_2$, 3 x 20 ml. The combined organic layers were dried over $Na_2SO_4$. The solution was filtered and the solvent was removed on a rotary evaporator. The product was purified by column chromatrography on 45 g of 70-230 mesh silica gel. The column was eluted with 3% MeOH/$CH_2Cl_2$. Fractions were pooled and the solvent removed on a rotary evaporator. The product was dried on a vacuum pump giving a clear colorless oil N-benyl-3S-acetamido-pyrrolidine 400 mg (40%). Mass Spectrum, m/z 218.

NMR ($CDCl_3$); delta value
2.20 (m, 2H)
2.30(s, 3H)
3.70 (m, 4H)

4.00 (m, 1H)
4.30 (dd, 2H, J = 6Hz, 15Hz)
6.30 (bs, 1H)
7.10 (m, 5H)

The 400 mg (1.8 mmol) of N-benzyl-3S-acetamido-pyrrolidine in 50 ml of MeOH was treated with 400 mg of 10% Pd/C and 1.8 ml in HCl under a hydrogen atmosphere at room temperature. The catalyst was removed by filtration. The solvent was stirred with 3g of Rohm and Haas' IRA 400 ion exchange resin. The resin was removed by filtration and was washed with MeOH 2x50 ml. The solvent was removed on a rotary evaporator. The product was dried on a vacuum pump giving 230 mg of a clear colorless oil 3s-acetamido-pyrrolidine. Mass Spectrum, m/z 128.

NMR ($CDCl_3$); delta value
2.0 (s, 3H)
2.1-3.0 (m, 6H)
4.2 (m, 1H)
4.6 (s, 1H)
7.3 (m, 1H)

In Vitro Tests

The in vitro antibacterial activity of the test compound was determined by conventional agar dilution procedures. The organisms were grown overnight in brain-heart infusion (BHI) broth (Difco 0037-01-6) at 36° C. Twofold dilutions of the stock solution (2000 mcg/ml) of the test compound were made in BHI agar to obtain a test concentration ranging from 200 to 0.005 mcg/ml. The plate was inoculated with approximately $10^4$ organisms. It was then incubated at 36° C for 18 h. The minimal inhibitory concentration was the lowest concentration of the test compound that yielded no visible growth on the plate.

The results of in vitro testing of Example 1 are shown in Table II below:

Table II

| In Vitro Anaerobes Test for Compound of Example 1 | | | |
|---|---|---|---|
| | | MIC (mcg/ml) | |
| BACTEROIDES | FRAGILIS | 105AT25285 | .4 |
| BACTEROIDES | FRAGILIS | 784 | .4 |
| BACTEROIDES | FRAGILIS | UC-2 | .8 |
| BACTEROIDES | FRAGILIS | SFM2906A | .4 |
| BACTEROIDES | FRAGILIS | SFM2975-7 | 1.6 |
| BACTEROIDES | FRAGILIS | SFM2929-1 | .8 |
| BACTEROIDES | SP | SFM2975-2 | 1.6 |
| BACTEROIDES | VULGATUS | 792 | .4 |
| BACTEROIDES | VULGATUS | SFBC 2375 | .4 |
| BACTEROIDES | DISIENS | ATCC 29426 | =<.05 |
| BACTEROIDES | THETAIOTAOMICRON | ATCC 29742 | .8 |
| BACTEROIDES | THETAIOTAOMICRON | 106 | .8 |
| BACTEROIDES | THETAIOTAOMICRON | ATCC 29741 | .8 |
| BACTEROIDES | LOESCHEII | ATCC 15930 | .8 |
| BACTEROIDES | BIVIUS | B 6140 | .4 |
| FUSOBACTERIUM | NUCLEATUM | ATCC 25586 | .8 |
| FUSOBACTERIUM | SP | GS-10 | .4 |
| VEILLONELLA | PARVULA | ATCC 10790 | .4 |
| CLOSTRIDIUM | PERFRINGENS | 104AT13124 | .1 |
| CLOSTRIDIUM | PERFRINGENS | SFBC 2026 | .2 |
| CLOSTRIDIUM | PERFRINGENS | 788 | .1 |
| CLOSTRIDIUM | DIFFICILE | ATCC 9689 | 1.6 |
| CLOSTRIDIUM | DIFFICILE | ATCC 17857 | .8 |
| CLOSTRIDIUM | RAMOSUM | 7 | =<.05 |
| PROPIONIBACTERIUM | ACNES | 132 | .8 |
| PEPTOCOCCUS | ASACCHAROLYTICUS | ATCC 14963 | .4 |
| PEPTOCOCCUS | MAGNUS | ATCC 29328 | .2 |
| PEPTOSTREPTOCOCCUS | SP | TB-11 | .4 |
| PEPTOSTREPTOCOCCUS | MICROS | ATCC 33270 | .2 |
| PEPTOSTREPTOCOCCUS | ANAEROBIUS | ATCC 27337 | .2 |

Experimental Anaerobic Abscess Model

B. fragilis (ATCC25285) was passaged three times in connective tissue air pouches of mice (CF-1 female mice weighing 20 g obtained from Charles River Breeding Laboratories, North Wilmington, MA), in order to obtain a virulent organism which consistently yielded viable counts greater than $10^5$ cfu in the air pouch over a seven day period. This culture was maintained frozen at -60°C. An 18 h culture of the passaged virulent B.fragilis in thioglycollate broth was diluted 1:2 in physiological saline. 0-5 ml portion of this diluted culture was used to inject the air pouches of mice.

Air pouches were made on the dorsum by the injection of 2-5 ml of air into subcutaneous tissue of mice as described by Clark & Weinhold, Journal of Medical Microbiology 12, 233-7, 1979. The inoculum was then injected directly into the air pouch.

During the development of this model, the connective tissue air pouch contents were removed from five mice daily, for five days, and cultured quantitatively on anaerobic blood agar. Air pouches infected with B. fragilis were filled with purulent fluid, whereas air pouches injected with air or saline contained no material.

Mice were treated subcutaneously. Groups of five mice were treated with at least three different doses of each of the test compounds. The drugs were administered 1 h after infection and followed by twice a day treatment for 4 days. The daily doses were administered 8 h apart. Five mice were left untreated to be used as controls.

Eighteen hours after the last treatment, the connective tissue air pouch and its contents were removed, homogenized and cultured quantitatively on anaerobic blood agar. The geometric means of viable bacterial

count in the abscess were calculated and compared to those in untreated mice.

The in-vivo experimental anaerobic abscess test results are shown in Table III below:

Table III

| Numbers of viable Bacteroides fragilis isolated from abscesses | | | | |
|---|---|---|---|---|
| Dose (mg/kg) | Log CFUs (+SEM) | | | |
| | Example 3 | Ciprofloxacin | Metronidazole | Untreated control |
| 0 | | | | 3.4 (±0.16) |
| 3.1 | 3.7 (±0.5) | 4.0 (±0.1) | 3.8 (±0.2) | |
| 12.5 | 2.7 (±0.5) | 3.6 (±0.2) | 3.4 (±0.1) | |
| 50.0 | 0 (±0.0) | 2.4 (±0.2) | 2.0 (±0.2) | |

As can be seen from Table III, the compound of Example 3 is 4 times more active than ciprofloxacin (another quinoline antibacterial) and 3-4 times more active than metronidazole against Bacteroides fragilis ATCC 25285. Metronidazole is a clinically useful antibacterial agent which is recommended for treatment of B. fragilis infections in man. Example 3 cleared the infection in all abscesses when treated with 50 mg/kg, whereas in ciprofloxacin treated animals 2.4 (±0.5) log CFUs were found in the abscesses, and in metronidazole treated animals 2.0 (+0.2) log CFUs were found in the abscesses.

Thus, the compounds of formula 1 are more active than ciprofloxacin and metronidazole. Since metronidazole is considered to be a very effective anti-anaerobic agent and the above tests show the compounds of formula I to be more effective than metronidazole, the compounds of formula I is considered an exceptionally effective anti-anaerobic agent in vivo.

**Claims**

**1.** Use of a compound having the formula I:

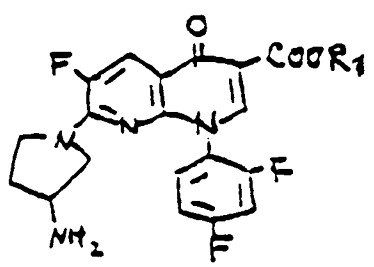

wherein $R_1$ is a hydrogen atom or a carboxy protecting group and pharmaceutically acceptable salts thereof, for preparing a drug useful in treating a bacterial infection caused by an anaerobic bacteria.

**2.** The use according to claim 1 wherein the compound is 1-o,p-difluorophenyl-6-fluoro-1,4-dihydro-4-oxo-7(s)-(3-aminopyrrolidin-1-yl)-1,8-naphthyridine-3-carboxylic acid and pharmaceutically acceptable salts thereof.

**Patentansprüche**

**1.** Verwendung einer Verbindung der Formel I

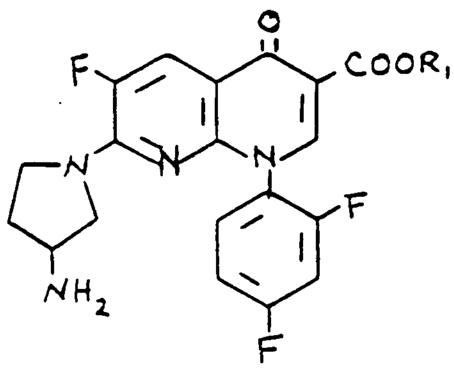

in der $R_1$ ein Wasserstoffatom oder eine Carboxyschutzgruppe bedeutet, und deren pharmazeutisch annehmbaren Salze zur Herstellung eines Arzneimittels, das für die Behandlung einer durch ein anaerobisches Bakterium verursachte Infektion brauchbar ist.

**2.** Verwendung gemäß Anspruch 1, worin die Verbindung 1-o,p-Difluorphenyl-6-fluor-1,4-dihydro-4-oxo-7-(S)-(3-aminopyrrolidin-1-yl)-1,8-naphthyridin-3-carbonsäure ist, sowie pharmazeutisch annehmbare Salze hiervon.

**Revendications**

**1.** Emploi d'un composé répondant à la formule I :

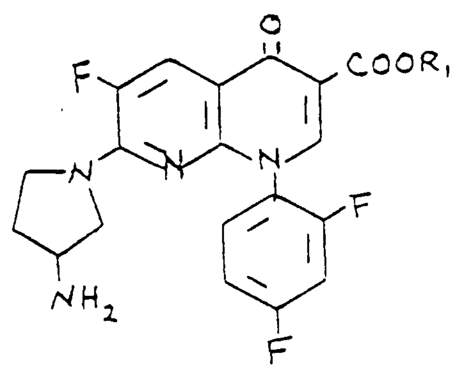

dans laquelle $R_1$ est un atome d'hydrogène ou un groupe carboxy-protecteur et ses sels pharmaceutiquement acceptables, pour préparer un médicament utile dans le traitement d'une infection bactérienne provoquée par une bactérie anaérobie.

**2.** Emploi selon la revendication 1, dans lequel le composé est l'acide 1-o,p-difluorophényl-6-fluoro-1,4-dihydro-4-oxo-7(S)-(3-aminopyrrolidine-1-yl)-1,8-naphtyridine-3-carboxylique et ses sels pharmaceutiquement acceptables.